# EUROPEAN PATENT SPECIFICATION

(11) **EP 3 294 399 B1**
(45) Date of publication and mention of the grant of the patent: **20.03.2024**
(21) Application number: 16793512.1
(22) Date of filing: 12.05.2016
(51) Int. Cl.: A61B 5/296, A61B 5/00, A61M 25/00

(54) **CATHETER SYSTEM FOR LEFT HEART ACCESS**
KATHETERSYSTEM FÜR LINKSATRIALEN ZUGANG
SYSTÈME DE CATHÉTER POUR ACCÈS AU COEUR GAUCHE

(30) Priority: 12.05.2015 US 201562160151 P; 19.05.2015 US 201514715788; 21.03.2016 US 201615075317
(43) Date of publication of application: 21.03.2018
(73) Proprietor: Acutus Medical, Inc., Carlsbad, California 92008 (US)
(72) Inventor: HASSETT, Jim, Eden Prairie, MN 55347 (US)
(74) Representative: Zimmermann & Partner Patentanwälte mbB
(86) International application number: PCT/US2016/032053
(87) International publication number: WO 2016/183300

(56) References cited:
- US-A1- 2004 044 350
- US-A1- 2005 159 738
- US-A1- 2007 021 648
- US-A1- 2007 270 751
- US-A1- 2013 274 784
- US-A1- 2013 304 051

## Description

### Background of the Invention

Many patients undergo diagnostic or interventional procedures in their left heart. For example, a patient with atrial fibrillation may undergo an electrophysiological study inside the chambers of the left heart to determine the physical location of the source of the arrhythmia. This may require the use of electrophysiology (EP) catheters positioned in side the left heart and in contact with the walls of the heart to make electrical measurements to determine the location and propagation properties of the arrhythmia. In some instances, a particular location may be an anatomic defect that can be ablated by yet another catheter system. In a similar fashion a patient may undergo left heart catheterization to receive a Left Atrial Appendage (LAA) Occlusion device that is placed in the LAA.

Although these procedures are becoming routine there is a need to improve the devices that allow the physician to gain access to the left heart from the right side of the heart and the venous system. The present standard of care involves the use of a stiff straight catheter to reach the right atrium (RA) from an entry site in the leg near the groin. Typically, the venous system is accessed in the groin via the familiar Seldinger procedure. With the conventional catheter placed in the RA a supplemental and exposed needle is advanced out of the conventional catheter and it is used to approach and pierce the septal wall dividing the right heart from the left heart.

This technique is cumbersome, requires a substantial amount of fluoroscopic exposure to both the patent and the physician and is potentially dangerous of several reasons.

The inventive devices, systems and methods of the present disclosure provide distinct improvements over the known techniques, in terms of ease of use, safety, and efficiency.

Other previously proposed arrangements are disclosed in US 2005/159738 (combined ECG probe and RF cutter/cauteriser), US 2004/044350, and US2007/270751.

US 2005/159738 relates to a surgical perforation device with electrocardiogram (ECG) monitoring ability and method of using ECG to position a surgical perforation device.

### Summary of the Invention

Claim 1 defines a system according to the invention.

Devices and systems of the present disclosure include a first (or inner) catheter assembly and two different outer catheter assemblies. The inner catheter is used with one of the two outer catheters and these two assemblies combined form a system for finding and crossing the fossa ovalis treating a patient according to the methods described herein.

The first or inner catheter assembly can be used with conventional catheters as well but is less effective and more cumbersome to use in that configuration.

The paired catheter systems are useful for carrying out a method of finding and crossing the fossa oval is between the right and left atriums of the heart. furhter

In the various configurations described herein, the first catheter assembly is coupled to one of the second or third catheter assemblies and form a cooperative system for carrying out steps in an electrographic location procedure. The first catheter assembly is supported by its companion outer catheter (second or third catheter assembly) and together they are used to electrically probe the septal wall surface to determine electrographically the location of the fossa ovalis (FO). The first catheter assembly includes an echogenic piercing tip that may be deployed to extends from the distal tip for piecing the FO. The distal tip is sufficiently opaque to x-rays to be seen radiographically and reflective enough to be visualized using ultrasound.

Therefore, in use the outer catheter assembly (in the form of either the second catheter assembly or third catheter assembly) supports and places the distal tip of the first catheter assembly at the wall of the septum. The first catheter carries an electrode that is electrically exteriorized to the proximal end of the first catheter. A electrical connection is available on the proximal end of the catheter that may be connected to a standard electromyography (EMG) recoding machine in a unipolar configuration. With the electrode tip within the outer sheath it can still pick up signals from the distal end of the catheter combination and the electrical activity may be observed as the assembly is tracked on the interior wall or septum of the heart. By dragging the distal end region of the system down the septal wall, the FO is characterized by the nature of the electromyography waveform signal. The magnitude and shape of the waveforms are distinct along the septum. When the His bundle signal is diminished that indicates the ideal location for crossing into the left heart. It is important to note that this procedure is carried out with the electrically conductive needle retracted, although the touching of the heart with the blunt catheter tip does cause the EMG to show a so called injury current.

With the specific FO location identified electrographically, and verified with another and different modality such a X-ray fluoroscopy, the first catheter assembly may be used cross the septum with a deployable needle, which also is extended from the distal tip. Once across the septum the second catheter assembly or third catheter assembly may be advanced into the left heart and used to approach the walls of the left atrium. When a desirable location is reached the first catheter assembly is uncoupled from the outer catheter assembly and the first catheter assembly is withdrawn.

With the desired treatment location found the first catheter assembly remains stationary and the septum is punctured with the same device via extension of the needle. Although complex electrically and electrographically, the system and method described is quicker and more accurate than the conventional blind probing that is the current state of the art.

### Brief Description of the Drawings

Fig. 1a-1d are several external views of a first catheter assembly.
Fig. 2a is a longitudinal section view of the catheter assembly shown in Figs. 1a-1d wherein a thumb slide and an electrode/needle distal assembly is shown in the retracted state.
Fig. 2b is a longitudinal section view of the catheter assembly of Fig. 2a. wherein the thumb slide and the electrode/needle distal assembly is shown in the extended state.
Fig. 2c is a side view of the embodiment shown in Figs. 2a-2b, wherein the assembly is shown connected to a connection lead and an EMG recording system/display.
Fig. 3a is a longitudinal section, detailed view of the distal end region of the catheter assembly shown in Figs. 1a-1d with a guidewire extending therethrough and the electrode/needle distal assembly shown in the retracted state.
Fig. 3b is a longitudinal section, detailed view of the distal end region of the catheter assembly shown in Fig. 3a wherein the guidewire is shown extending therethrough, and with the electrode/needle distal assembly shown in the extended state.
Fig. 3c is a longitudinal section, detailed view of the distal end region of the catheter assembly shown in Fig. 3d with the guidewire removed and the electrode/needle distal assembly shown in detail and in the extended state.
Fig. 4 is a cross-sectional view of the distal end region of the catheter assembly shown in Figs. 1-3c.
Fig. 5a is a rear perspective view of the first catheter assembly engaged to a second catheter assembly.
Fig. 5b is a rear perspective view of the first catheter assembly and second catheter assembly shown in Fig. 5a, but shown prior to their engagement so as to illustrate their proper alignment for engagement.
Fig. 5c is a rear perspective view of the first catheter assembly and second catheter assembly shown in Fig. 5a and 5b, wherein improper alignment for engagement is illustrated.
Fig. 5d is a detailed perspective view showing the proper alignment and function of the engagement mechanisms of the first catheter assembly and second catheter assembly shown in Figs. 5a and 5b.
Fig. 6 is a detailed longitudinal section view of the proximal regions of the first catheter assembly and second catheter assembly shown in Fig. 5a.
Figs. 7a-7e are several external views of the second catheter assembly shown in Figs. 5a-6.
Fig. 8 is a longitudinal section view of the proximal region of the second catheter assembly (such as is also shown in Fig. 6, but it is shown here without the first catheter assembly engaged thereto).
Figs. 9a-9e are several external views of a third catheter assembly.
Fig. 10a is a rear perspective view of the first catheter assembly engaged to the third catheter assembly.
Fig. 10b is a rear perspective view of the first catheter assembly and third catheter assembly shown in Fig. 10a, but shown prior to their engagement, so as to illustrate their proper alignment for engagement.
Fig. 10c is a rear perspective view of the first catheter assembly and third catheter assembly shown in Fig. 10a and 10b, wherein improper alignment for engagement is illustrated.
Fig. 11a is detailed top down view of the handle of the third catheter assembly with the control knob activation button shown in an unactuated or un-pressed state.
Fig. 11b is detailed top down view of the handle of the third catheter assembly with the control knob actuation button shown in an actuated or pressed state.
Fig. 11c is a sectional view of the handle of the third catheter assembly with the control knob in a neutral or un actuated state.
Fig. 11d is a sectional view of the handle of the third catheter assembly with the control knob shown in a rotated state.
Fig. 12a. is a top down view of the third catheter assembly shown in a neutral state.
Fig. 12b. is a longitudinal section view of the third catheter assembly shown in Fig. 12a.
Fig. 12c. is a top down view of the third catheter assembly shown in a fully actuated state wherein the control knob is turned to fully actuate the distal end region of the assembly whereby it is turned 180 degrees back on itself.
Fig. 12d. is a longitudinal section view of the third catheter assembly shown in Fig. 12c.
Fig. 13 is a detailed view of the distal end region of the third catheter assembly showing the manner and degree of its possible articulation relative to a neutral position, such as is shown in Figs. 12a-12d.
Fig. 14 shows an embodiment of the invention in use during a procedure wherein the distal end region of the first catheter assembly extends past the distal end region of the third catheter assembly during initial insertion of the system into a patient's heart.
Fig. 15 shows an embodiment of the invention in use during a surgical procedure wherein the distal end region of the first catheter assembly is manipulated and drawn along the superior vena cava so as to align the electrode/needle distal assembly with the fossa ovalis.
Fig. 16 shows a representative electro-gram typically registered when the distal end region of the first catheter assembly is in the position shown in Fig. 15.
Fig. 17 shows the distal end region of the first catheter assembly properly positioned adjacent to the fossa ovalis during the procedure depicted in Fig. 15.
Fig. 18 shows a representative electro-gram typically registered when the distal end region of the first catheter assembly is in the position shown in Fig. 17.
Fig. 19 represents a real time recording of the electrical activity (via electromyography) detected when the system is in the position shown in Fig. 17.
Fig. 20 and 21 show the time domain representative of the exploratory motions of the distal end region of the first catheter assembly shown in Figs. 15 and 17.
Fig. 22 shows characteristic electro grams associated with different regions of the left atrium detected by the electrode/needle distal assembly of the first catheter assembly when immediately adjacent to the respectively depicted regions.

### Detailed Description of the Invention

### Inner Catheter or First Catheter Assembly

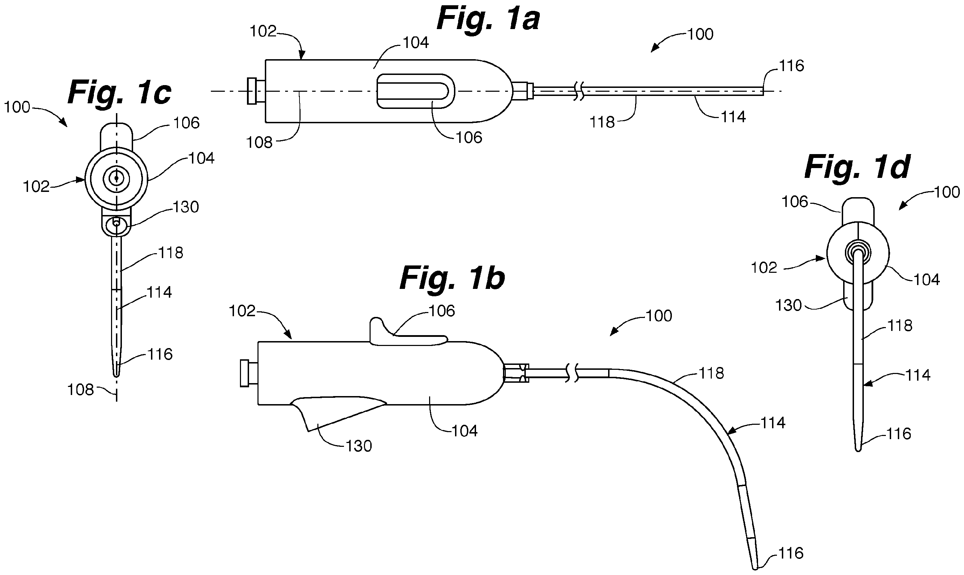

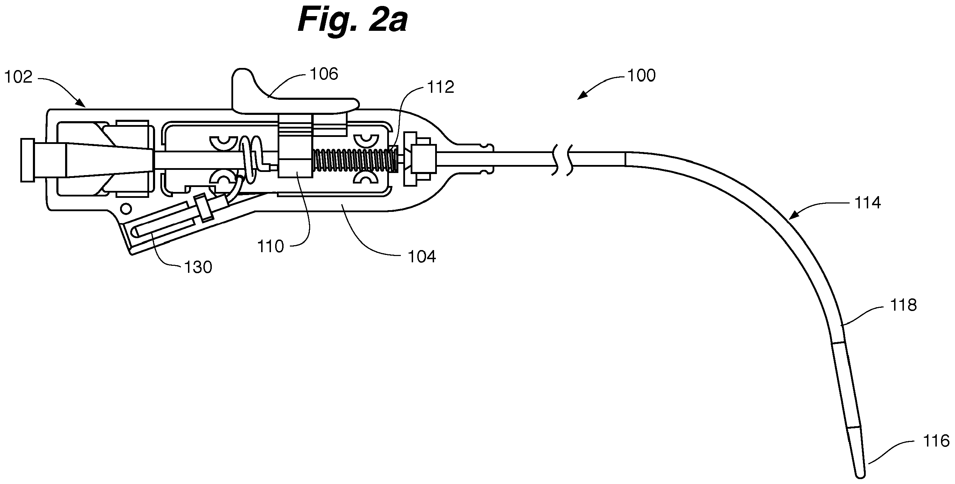

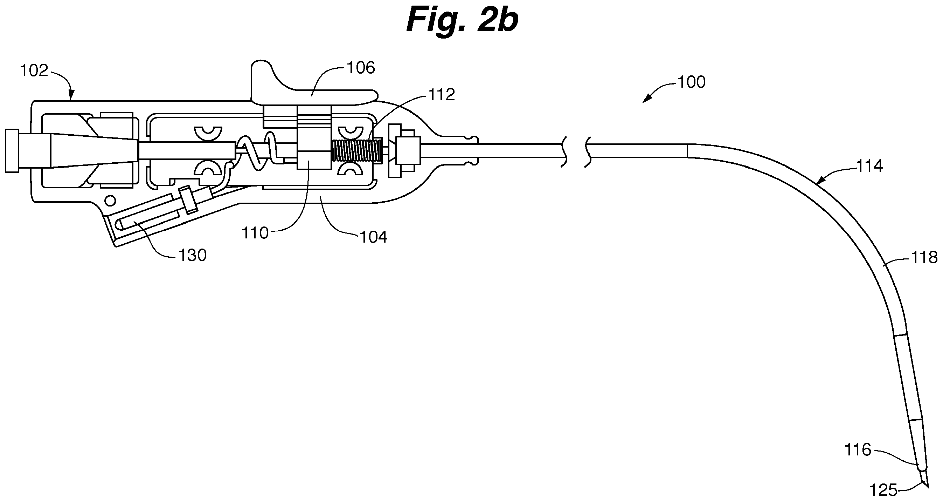

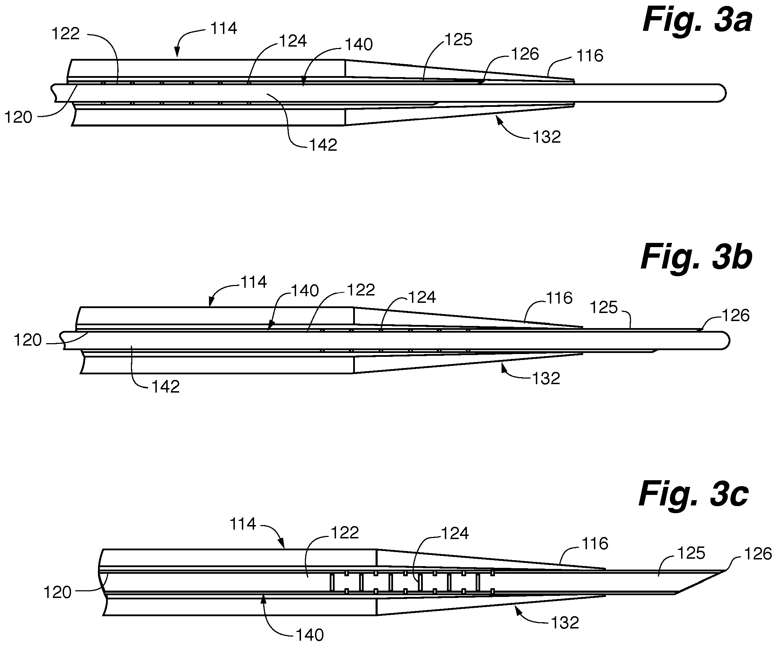

Turning to Figs. 1a-1d a first catheter assembly generally designated 100 is shown. At a proximal end region 102 there is user interface handle 104. A thumb operated slide 106 is carried in the handle 104 and adapted for sliding motion along the axis 108 of the first catheter assembly. The thumb slide 106 is mechanically engaged to an electrode/needle assembly 125 that is shown in Figs. 2b and 3a-3c and contained within the distal end region 114 of the first catheter assembly 100 when the thumb slide 106 is in the unactuated state shown in Figs. 1a-1d and 2a, for example.

As is best shown in Figs. 2a and 2b, in operation, the thumb slide 106 forces a tang 110 to compress a spring 112 located along and concentric with the axis 108. Motion of the thumb slide 106 toward the distal end region 114 of the first catheter assembly 100 causes the electrode/needle assembly 125 (Fig. 2b) to emerge from the distal tip 116 of the distal end region casing (or housing) 118, as seen in Fig. 2a and Fig. 2b respectively. The mechanical interface between the electrode/needle assembly 125 and the tang 110 may be a wire, shaft, hypo-tube or other elongate member which extends distally from the handle 104, through the casing 118.

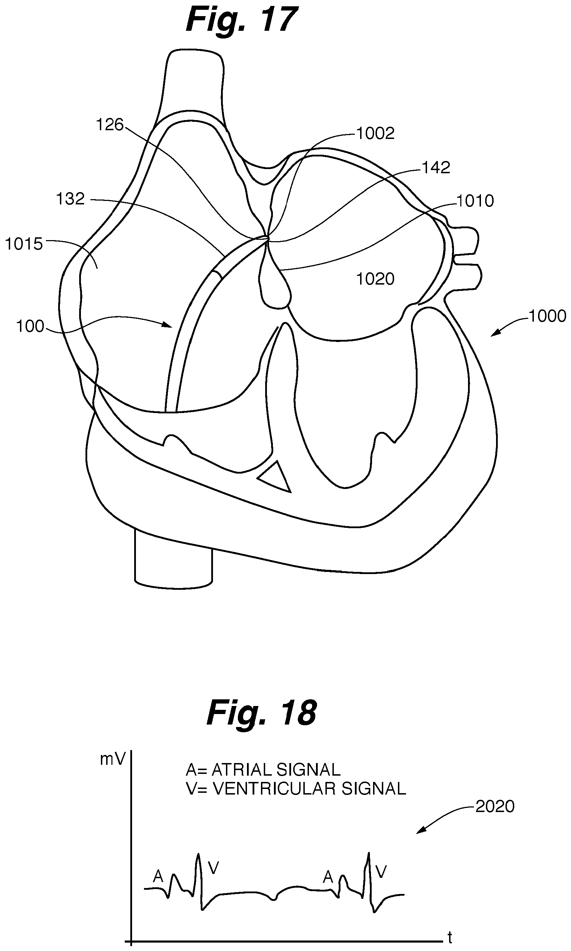

As may be seen in Figs. 3a-3c, the distal assembly or distal end region 114 has several important features. A hypo-tube 122 has series of laser-machined partially circumferential slits or openings, typified by slit 124 shown in Figs 3a-3C, which cooperate together to render the distal end region 114, and the distal tip 116 especially, flexible in any direction or plane and be compliant with the shape of a companion outer catheter (features of which are shown and discussed elsewhere in this disclosure). The needle/electrode 125 includes a piercing tip 126. This tip is electrically coupled via wire 120 to the electrical connector port 130 (shown in Figs. 1-2). The distal assembly casing 118 tapers to a small diameter at the distal tip 116 and serves as a dilation surface 132; whereby when the distal tip 116 is advanced into the heart 1000 and through the wound cite (opening) 1002 in the septum 1010, created by the piercing tip 126; the dilatation surface 132 acts to open the wound cite 1002 further to allow the catheter assembly 100 better access into the left atrium 1020, from the right atrium 1015, such as is depicted in Fig 17 and discussed in greater detail below.

Returning to Figs 3a-3c, the hypo-tube 122 and needle/electrode 125 also define a central guidewire lumen 140 through which a guidewire 142 is positioned to aid in advancing the catheter assembly 100 (and the joined multiple catheter assembly system 500 discussed in greater detail below) to the treatment cite.

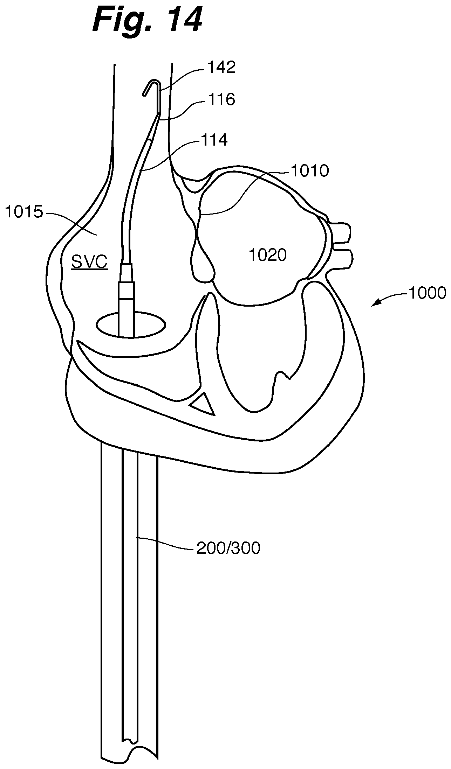

In Fig. 3a the first catheter assembly 100 is shown with the needle/electrode 125 in the retracted position with the guidewire 142 in place within the lumen 140. Such a configuration is representative of how the assembly 100 is arranged during advancement through the vascular anatomy along the guidewire 142 and into the right atrium 1015 of the heart 1000 such as is shown in FIG. 14.

In Fig. 3b the needle/electrode 125 is shown in the extended position, wherein it extends out of the casing 118 and beyond the distal tip 116 of the first catheter assembly 100, with the guidewire 142 still in place.

In Fig. 3c, the guidewire 142 has been proximally withdrawn through the lumen 140 to allow the needle/electrode 125 unimpeded access to the septum 1002 such as in the manner shown in Fig. 17.

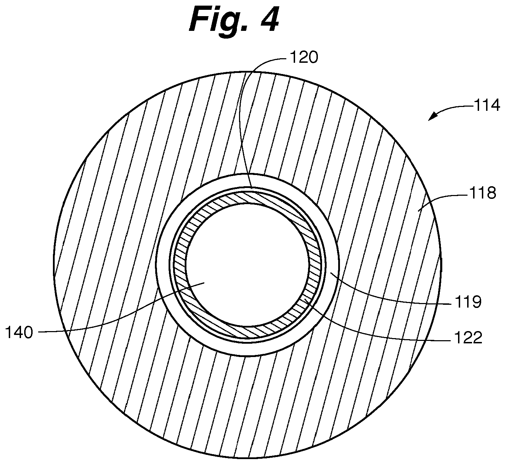

Fig. 4 shows a cross-sectional view of the distal end region 114 components including the housing or casing 118, the wire 120, the the hypo-tube 122 and guidewire lumen 140. An inherent feature of this arrangement is that the casing 118 defines a hypo-tube lumen 119 in which the hypo-tube 122 (and the distal end portion of which is the needle/electrode 125) is moveable (retraction and extension via thumb slide 106 discussed above) therein.

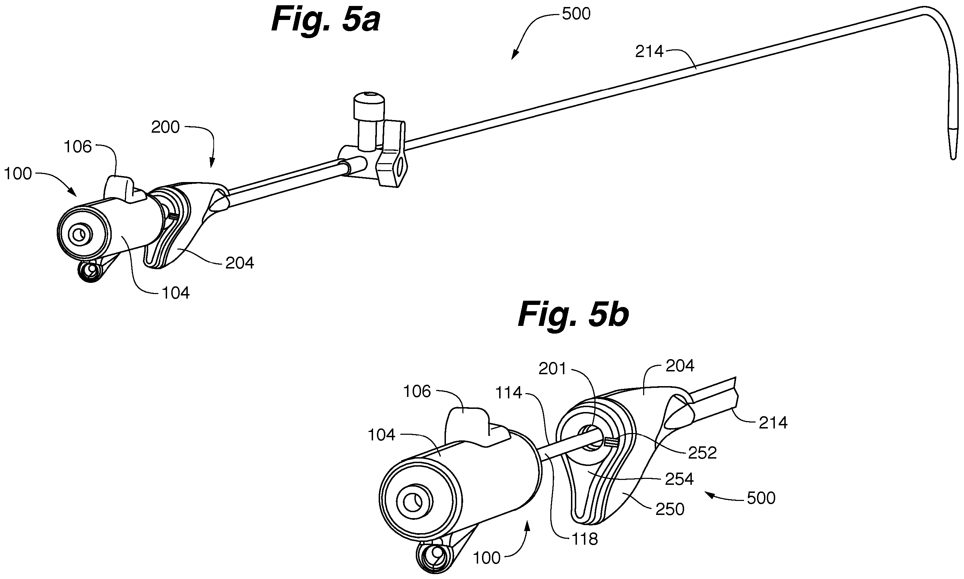

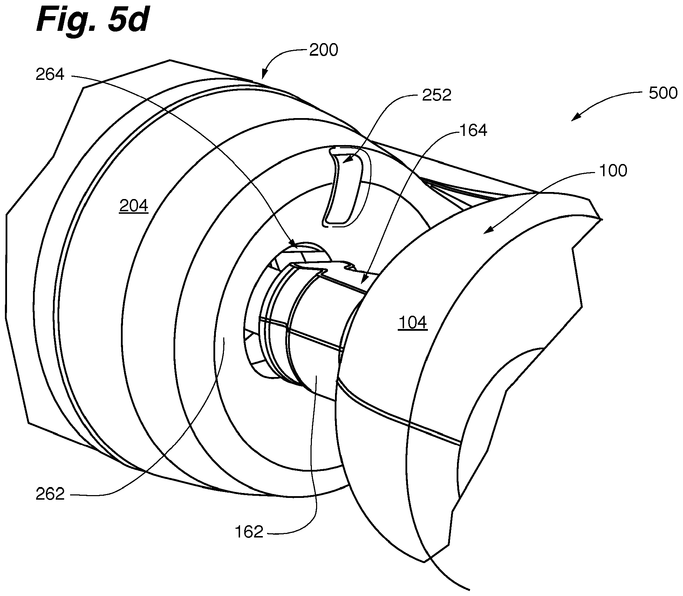

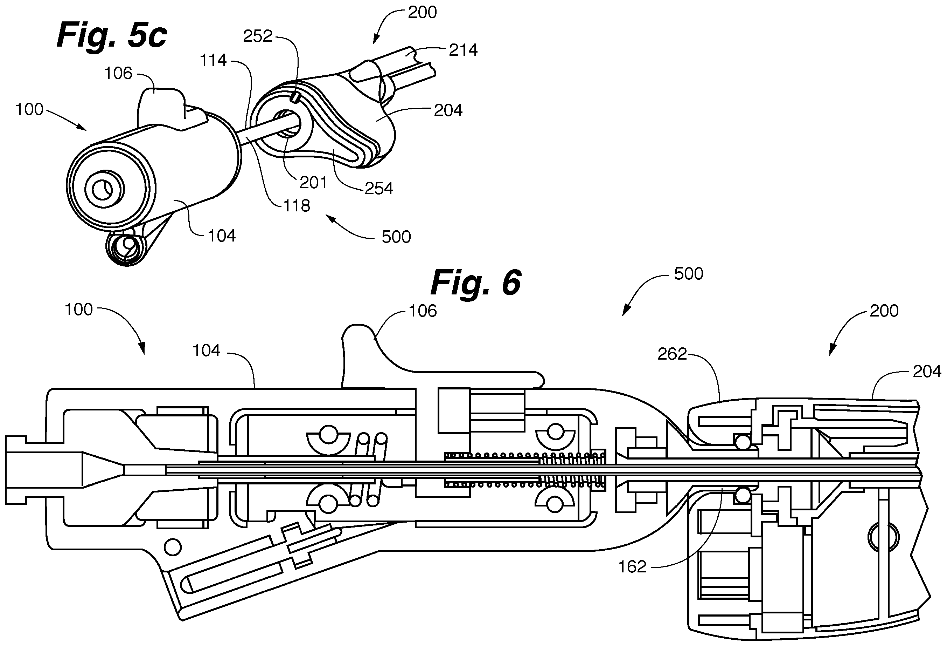

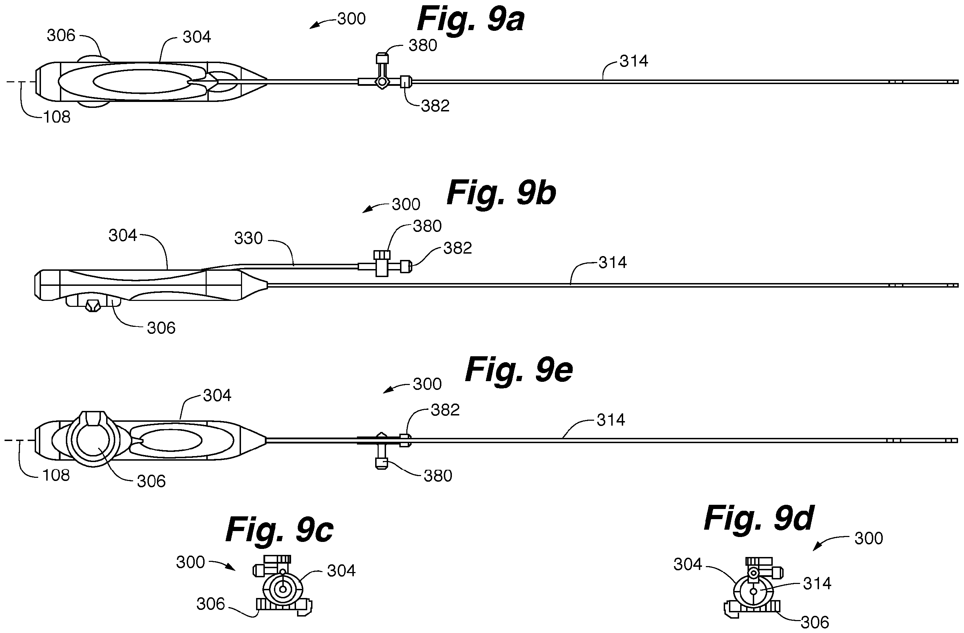

As mentioned above, in at least some embodiments the first catheter assembly 100 is the "inner" catheter of a multiple catheter system 500 wherein one of two types of "outer" catheters are used in conjunction there with. Such outer catheter assemblies and their manner of use with the first catheter assembly 100 are shown in Figs. 5-13 and are discussed below. For simplicity the two types of "outer" catheters are identified as a second catheter assembly 200 (shown in detail in Figs. 5-8) and a third catheter assembly 300 (shown in Figs. 9-13) respectively.

### Outer Catheter Option One -Second Catheter Assembly:

Fig. 5a shows the distal end region 114 of the first catheter assembly 100 that has been inserted into the second catheter assembly 200. The handle 104 of the first catheter assembly is coupled to the handle 204 of the second catheter assembly 200 by advancing the entire casing 118 of the distal end region 114 of the first catheter assembly 100 into and through a receiving lumen 201 defined by the handle 204 and distal end region 214 of the second catheter assembly 200, in the manner show in Fig. 5b, until the handles 104 and 204 are properly engaged and locked together in the manner described below.

In Fig. 5b an embodiment of a system 500 is shown wherein various mechanism are provided to ensure proper coupling between the handles 104 and 204. For example, the relative shapes of the handles 104 and 204 provide a natural aligning feature, whereby the narrower bottom portion or torque handle 250 of the handle 204 is longitudinally aligned with the protrusion of the connector port 130 of the handle 104. Another alignment mechanism is the presence of a visual guide or indicator slot 252 present on the distal surface 254 of the handle 204. This slot 252 provides a user with a visual guide whereby a corresponding protrusion (not show) on the handle 104 engages the slot 252 as the first catheter assembly 100 is coupled to the second catheter assembly 200 in the manner shown in Fig. 5b. If the proper longitudinal alignment between the handles 104 and 204 is not achieved, such as is depicted in Fig. 5c, the assemblies 100 and 200 cannot be properly coupled. Finally, a third mechanism may be provided such as is shown in Fig, 5d. In the embodiment shown in Fig. 5d, a direct coupling mechanism 260 is provided whereby an engagement shaft 162 of the first catheter assembly 100 is received into an end cap assembly 262 of the second catheter assembly 200. The engagement shaft 162 and end cap assembly 262 may respectively include any of a variety of structural protrusions, indentations or similar features to provide a "snap fit" and/or "lock and key" style interface between the two handles 104 and 204. In the specific embodiment shown the end cap assembly 262 includes a flat "rib" 264, which a correspondingly shaped groove 164 on the engagement shaft 162 slides over and receives so as prevent any relative rotational movement between the coupled first and second catheter assemblies. A detailed longitudinal sectional view of the first catheter assembly 100 and second catheter assembly 200 being properly aligned and coupled together to form a system 500 is shown in Fig. 6.

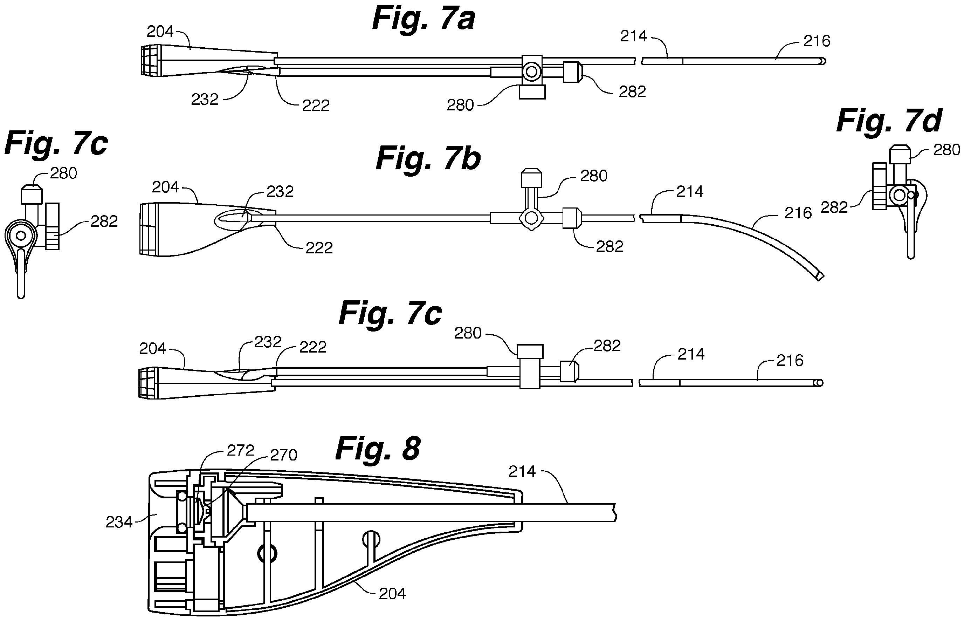

Referring now to the second catheter assembly 200 in more detail as depicted in Figs 7a-7e and in the sectional view of the handle 204 of Fig. 8, there is shown the entire second catheter assembly 200, which is also known as a guiding vascular introducer device comprised of a distal tubular section 214 that traverses through the handle 204. The distal tubular section 214 has a curved tip section 216. The handle 204 is further comprised of a side port tube 230. The external part of the side port tube 230 is located at the distal end of the handle 204 as shown best in Figs 7a, 7b and 7e. In these same figures there is shown a strain relief 222 at the junction of the distal tubular section 214 and handle 204 as well as a canted pass-through aperture 232 for the side port tube 230 to enter the handle 204.

The construction details of the invention are selected such that the useable length of the distal tubular section 214; including its curved tip section 216, shall be sufficient to reach from a patient's vascular insertion site, in the groin area, to the left atrium of their heart, typically 50 to 75 centimeters, but may be longer in taller patients. The inner diameter of the distal tubular section 214, including its curved tip section 216, shall be sufficient to accommodate various catheter devices, typically 5 French (1.65 mm) to 12 French (3.96 mm). The distal tubular section 214, including its curved tip section 216, shall be made of a medical grade polymer and may include wire braiding within its wall. The distal tubular section 214, including its curved tip section 216, may have coatings on its patient-contacting surfaces to provide lubricity and/or deter the formation of blood clots.

The side port tube 230 shall be made of a medical grade polymer and have an external length of approximately 5 to 20 centimeters. The handle 204 shall be a length sufficient to efficiently manipulate the introducer with the thumb and 3-5 fingers, typically between 3-5 centimeters. Furthermore, the handle 204 shall be of shape that provides an intuitive directional indicator (as discussed above) that is in plane with the curved tip section 216. One such shape is an inverted teardrop, as depicted in Figs. 5a-5c. The handle 204, including the canted pass-through aperture 232, shall be made of one or more medical grade thermoplastics such as polycarbonate, polyethylene, or nylon.

With specific regard to Fig. 8, within the handle 204 is shown a catheter access port 234. Of note, the side port tube 230 and distal tubular section 214 exit from the handle 204 in a parallel orientation (as is shown in Fig. 7b-7d). Port 234 includes a hemostasis valve housing 270 and mounting stem 272. The hemostasis valve housing 270 and integral mounting stem 272 are made of a medical grade thermoplastic such as polycarbonate, polyethylene, or nylon. The distal tubular section 214 is connected to the hemostasis valve housing 270 via injection molding or medical grade adhesive. The entire valve housing 270 shall be contained internally within the handle 204. The side port tube 230 is connected to the mounting stem 272 via medical grade adhesive.

Side port tube 230 include an access valve or stop-cock 280 along with an ancillary engagement port 282. Via this port and valve, various ancillary devices may be employed in conjunction with the secondary catheter assembly such as infusion pumps, drug delivery systems, and other diagnostic or therapeutic tools.

The advantages of the present invention include, without limitation, is that it allows the operator to efficiently torque the second catheter assembly 200 during a procedure. Typically, the operator only has a small hemostasis valve housing to serve as a torque handle. Furthermore, by removing the side port tube from the primary area of device manipulation eliminates the risks of interfering with operation and entangling with, and possibly dislodging, an adjacent device. Finally, the addition of a biomimetic coating on the patient-contacting surfaces with mitigate the risks of thrombogenesis, or the production of blood clots, which may lead to such adverse effects as stroke, myocardial infarction, or pulmonary embolus, all of which may be fatal.

In broad embodiment, the present invention is a guiding vascular introducer designed with an ergonomic torque handle with features that promote efficient and an improved safety profile.

### Outer Catheter Option One -Third Catheter Assembly:

Figs. 9a-9e illustrate various views of the second outer catheter option mentioned above, and hereinafter referred to as the third catheter assembly 300. The third catheter assembly 300 includes a proximal handle 304 and a distal end region 314. A side port tube 330 with a stop-cock 380 and ancillary engagement port 382 is also included in the third catheter assembly 300 and is used in the same manner for connecting ancillary systems and devices to the catheter, as the corresponding structures of the second catheter 200 assembly discussed above.

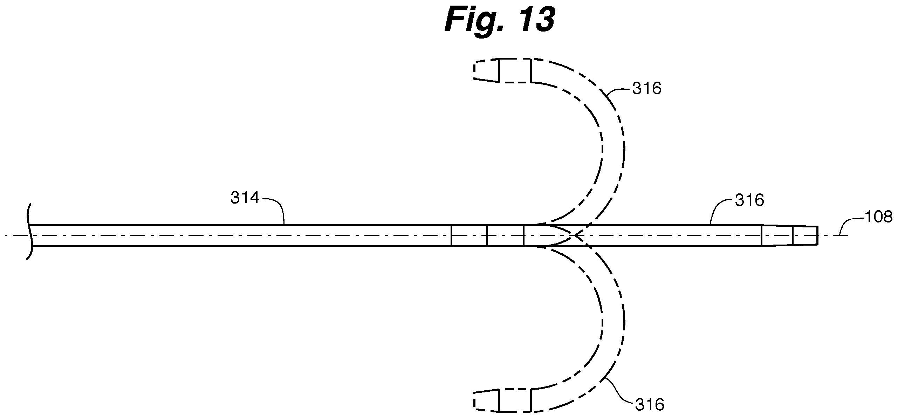

The third catheter assembly includes with the handle 304 a control knob 306 which is mechanically engaged to the distal tip 316 of the distal end region 314, whereby when the knob 306 is turned (by a user) the distal tip 316 moves relative to the longitudinal axis 108 of the distal end region 314 a specified distance and angle in the manner depicted in FIG. 13.

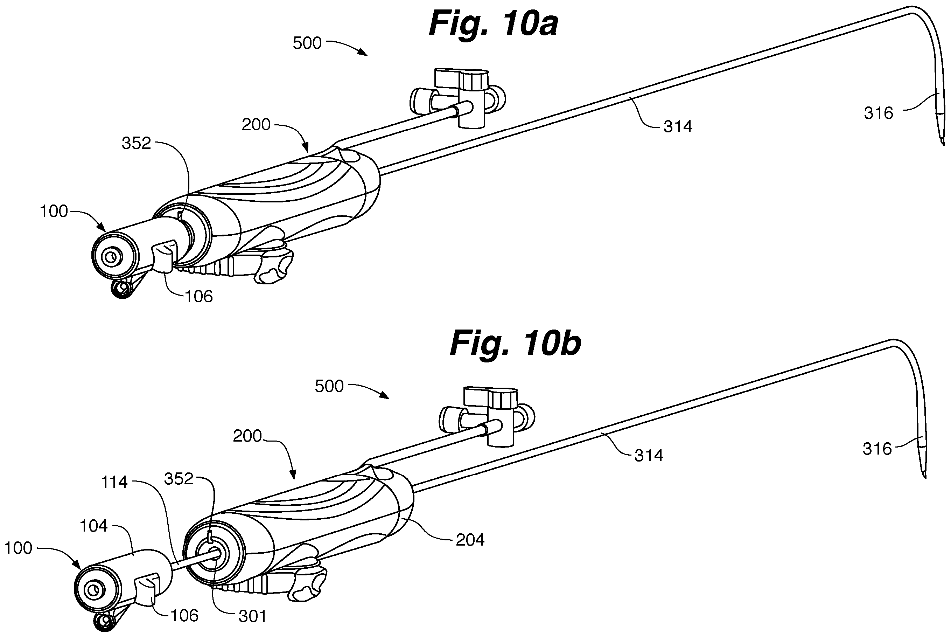

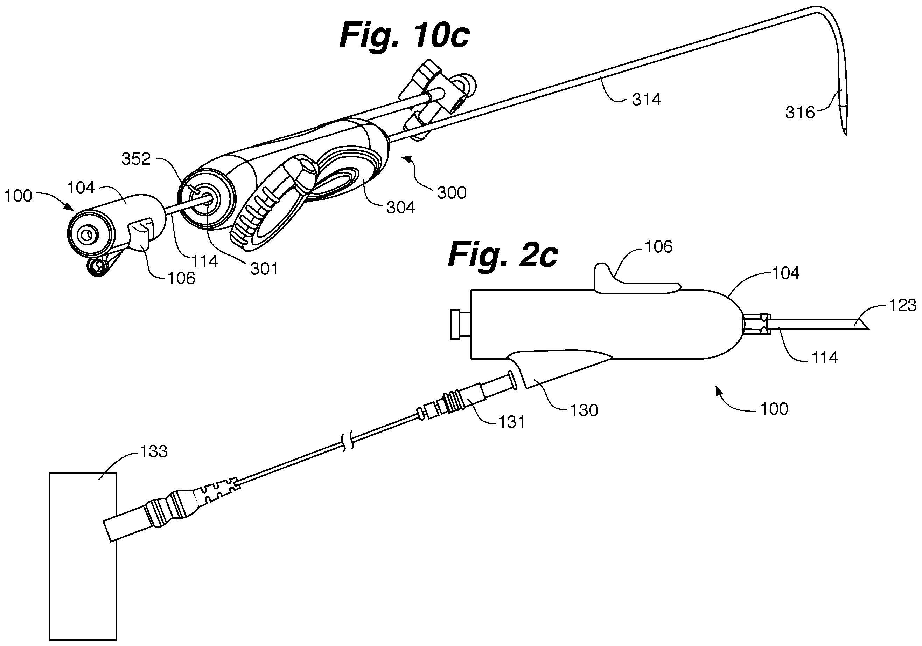

In the same manner as is shown in Figs. 5a-5c between the second catheter assembly 200 and the first catheter assembly 100, alignment between the third catheter assembly 300 and the first catheter assembly 100 must be achieved so as to allow their respective handles 104 and 304 to be coupled together such as in the manner depicted in Fig. 10b and 10a, so as to form a system 500. When improperly aligned, such as in the manner shown in Fig. 10c, the handles 304 and 104 are incapable of being coupled together. Proper alignment of the handles 304 and 104 may be be the same sort of mechanisms described in Figs 5a-5c above. In the embodiments shown in Figs. 10a-10c for example, the handle 304 includes a visual and mechanical guide in the form of an engagement slot 352 with which a user simply lines up the thumb slide 106 of the handle 104. If properly aligned, the slot 352 of the handle 304 will receive a protrusion or other feature (not shown) on the handle 104 to ensure proper coupling of the two handles 104, 304 when the first catheter assembly 100 is inserted into the lumen 301 of the third catheter assembly 300 in the manner shown in Fig. 10b.

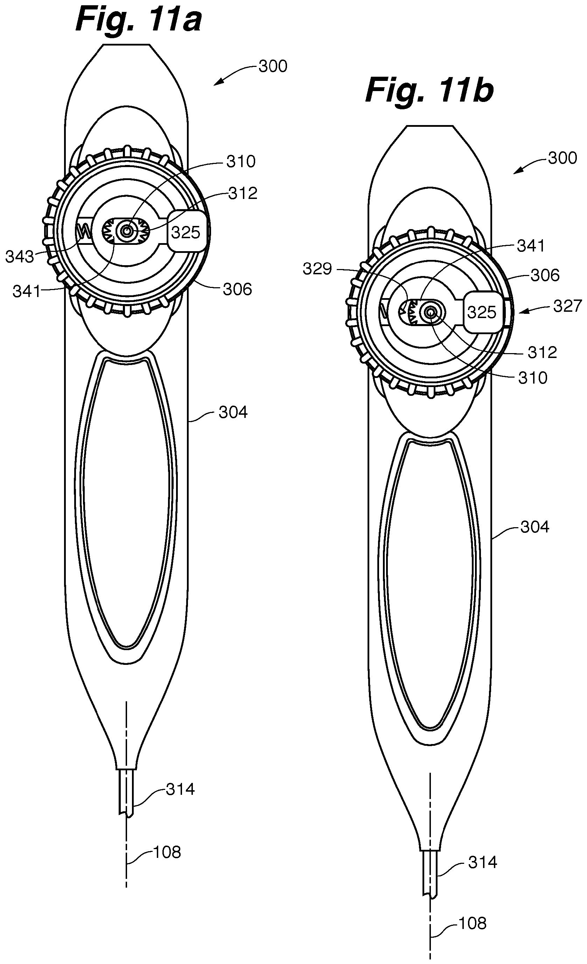

Turning now to the specifics of the third catheter assembly 300, as is best shown in Figs. 11a-11d, the third catheter assembly distal end region 214 extends from the distal end of handle 304 (and which receives the distal end region 114 of the first catheter assembly 100 therein) while the control knob 306 is located near the proximal end of the handle. The control knob turns on a control axis 310 defined by axel 312 orthogonal to the third catheter assembly's longitudinal axis 108.

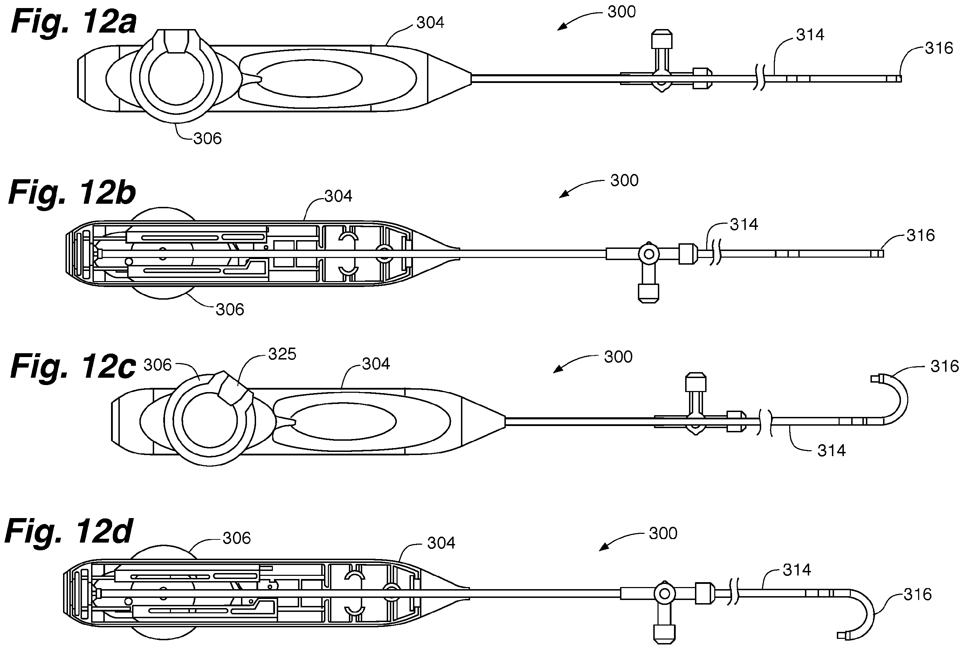

In use the physician turns the control knob 306 with his left hand and uses the thumb of the left hand to activate the control button 325. When this button is depressed as in the direction depicted at ref numeral arrow 327 the tooth 329 disengages from lock pinion gear 341. In the depressed or activated state (shown in Fig. 11b) the motion of the knob 306 is unlocked and the control knob 306 may be turned to steer or flex the distal tip 318 of the device. When control button 325 is released the tooth 329 is urged, by spring pressure of compression spring 343, back into position against the gear 341 to lock the knob's motion (and thus the position of the distal tip 316 as may be seen in Figs. 12-13) in place.

Figs. 12a-12d show the third catheter assembly 300 wherein the knob 306 is at rest or unactuated (Fig. 12b) and is fully actuated in a first direction (Fig. 12d). As can be seen, this activation and rotation of the control knob 306 causes the highly flexible distal tip 316 of the assembly to be drawn in different directions depending on the direction and extent that the control knob 306 if rotated. The flexible nature and degree of the distal tip's movement relative to the longitudinal axis 108 is shown in more detail in Fig. 13.

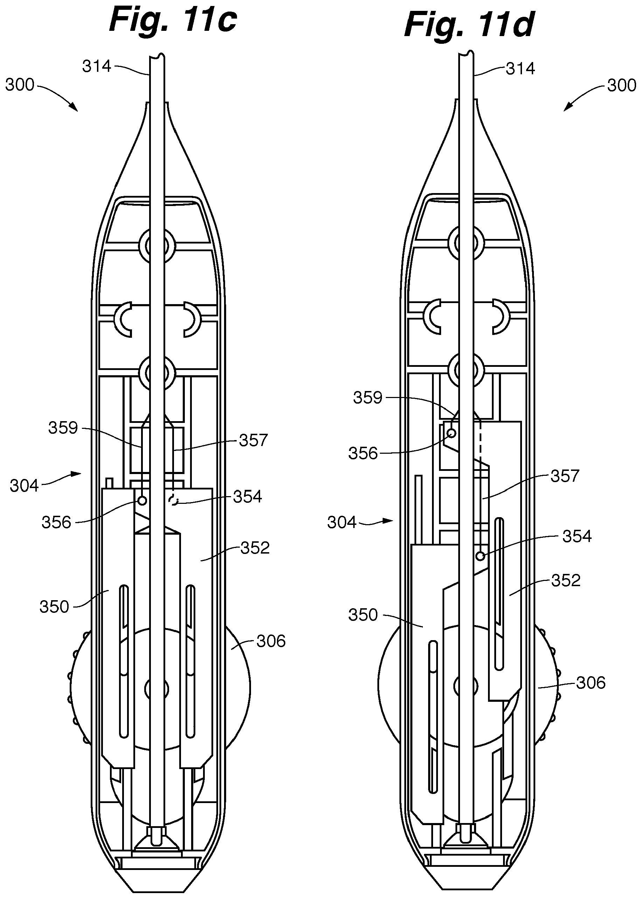

In the embodiments shown, the particular arrangement of components which allows the distal tip 316 to move in the manner described above is shown in more detail in the sectional views of Figs 11c-11d. As can be seen in these images, the pinion gear 341 (shown in Figs 11a and 11b) engages both rack 350 and rack 352. Rotation of the pinion gear 341 (via actuation of the button 325 and rotation of the knob 306 as described above) drives the racks 350 and 352, with each rack driven in the opposite direction. Cable anchor 354 and cable anchor 356 are moved with respect to each other providing traction to the pulls wires 357 and 359 (partially shown, and which extend distally to the distal tip) that deflect the deflectable distal tip 316 through an arc in a plane as depicted in Fig. 13.

Figs. 12a-12b show the deflectable distal tip 316 in its un-deflected state corresponding to the rack positions seen in Fig. 11c. Figs. 12c-12d show the deflectable tip 316 moving through a 180 arc driven by pull wire 357 and pull wire 359, each connected to its respective cable anchor 354 or 356. This curvature corresponds to the rack positions seen in Fig. 11d.8. Figs. 10a-10c shows an intermediate position corresponding to a deflection of approximately 90 degrees.

The construction details of the invention as shown in the preceding figures are that the useable length of the distal tubular section 314 shall be sufficient to reach from a patient's vascular insertion site, in the groin area, to the left atrium of their heart, typically 50 to 75 centimeters, but may be longer in taller patients. As is well known only the proximal and distal section of the catheters illustrated to facilitate disclosure of the invention and the inventive features in the most proximal and distal areas of the catheters. The inner diameter of the distal tubular section 314 shall be sufficient to accommodate various catheter devices, typically 5 French (1.65 mm) to 12 French (3.96 mm). The distal tubular section 314 shall be made of a medical grade polymer and may include wire braiding within its wall. The distal tubular section 314 may have coatings or a biomimetic surface on its patient-contacting surfaces to provide lubricity and/or deter the formation of blood clots. The side port tube shall be made of a medical grade polymer and have an external length of approximately 5 to 20-centimeters. The control knob 306 may be configured as a rotatable wheel, rotatable coaxial collar, slide, or lever.

### Method of Use

The various combinations of catheter assemblies 100, 200 and/or 300 as shown and described above, are (as has been mentioned) to be utilized as a system 500 for conducting a method of accessing the left heart from the right heart following advancement of the system 500 through the vasculature of a patient

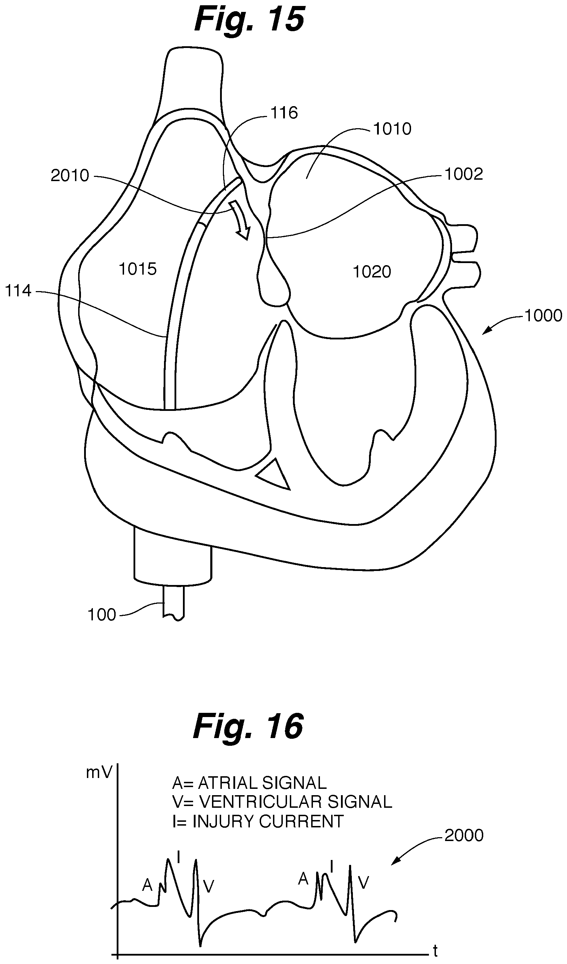

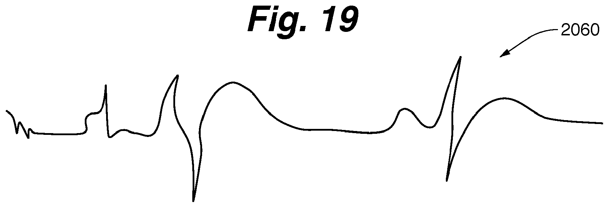

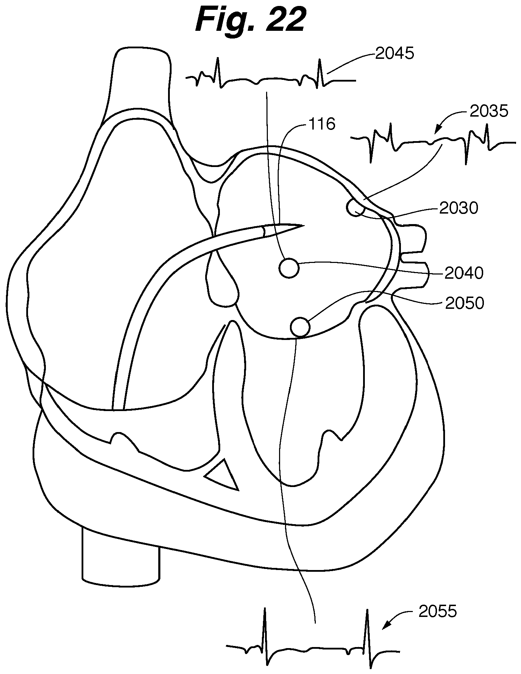

For example, the following stepwise sequence can be used to carry out the method not being claimed but useful for understanding the invention:
1. A physician or technician uses the Seldinger procedure to gain access to the femoral vein with a conventional needle puncture.
2. A long guidewire 142 is inserted through the needle and advanced under fluoroscopic guidance to the superior vena cava (SVC) such as is depicted in Fig. 14. As seen in Fig. 14 the guidewire 142 extends out of the distal tip and the location above the SVC is confirmed fluoroscopically. A small amount of contrast agent may be injected into the heart to visualize and confirm the location above the SVC.
3. Next, withdraw the needle over the wire leaving the wire 142 in place.
4. As seen in Fig. 15, the first catheter and third catheter assembly or first catheter assembly-second catheter assembly system 500 is advanced to the heart 1000 over the guidewire 142 and to the SVC.
5. Pull the guidewire 142 into the first catheter assembly.
6. Rotate the first catheter assembly-third catheter assembly or first catheter assembly-second catheter assembly system 500 to point medial as to be perpendicular to the plane of the interarterial septum 1010.
7. Connect an extension lead 131, such as is shown in Fig. 2c, between the connector port 130 of first catheter assembly 100 and an EMG recording system 133 to display unipolar signal from the needle/electrode 125 of first catheter assembly 100. In general a Wilson central terminal technique is used to provide the ground reference for the unipolar system. In this technique several surface electrode patches on the patient are taken collectively as the ground reference.
8. Maintaining system alignment by monitoring the system 500 via fluoroscopic imaging, electro gram and/or optional ultrasound imaging to locate the fossa ovalis 1002 such as is depicted in FIG. 15 by pulling the catheter assembly down along a path indicated by motion arrow 2010 while observing the electro gram shown in Fig. 16 where the characteristic wave form of the high septum location is seen at reference numeral 2000.
9. Once the fossa ovalis location has been reached as seen in Fig. 17, as confirmed by the characteristic waveform 2020 seen in Fig. 18 the physician is ready to pierce the heart wall. This is achieved by holding the system securely and actuate the thumb lever 106 to advance the piercing tip 126 through the fossa ovalis.
10. Optionally confirm presence in the left atrium via contrast injection (via side access ports 282/382 as previously shown and described) of pressure recording, and advance the guidewire 142 into the left atrium 1020, such as in the manner shown in Fig. 17.
11. Release the thumb lever 106 automatically retracting the piercing tip 126 under the force supplied by spring 112.
12. Advance system 500 into the left atrium 1020 while monitoring the electro gram which will have the form of the characteristic waveform 2060 seen in Fig. 19. Fig. 22 shows the catheter assembly roving in the left heart with chacteristic waves forms shown as taken from locations depicted as a circle 2030 high on the atrial wall showing a wave form 2035, while location circle 2050 is a location near the valve structures resulting in a characteristic wave form 2055. Location circle 2040 is corresponds to floating in the chamber and its wave form is seen at 2055.
13. Holding the system securely release and uncouple first catheter assembly and push sheath toward tip of first catheter assembly.
14. With the sheath near the wall of the atrium The first catheter assembly is withdrawn form the sheath, and the sheath is aspirated and flushed with heparinized saline. The sheath is now placed for the desired intervention such as ablation or device placement.

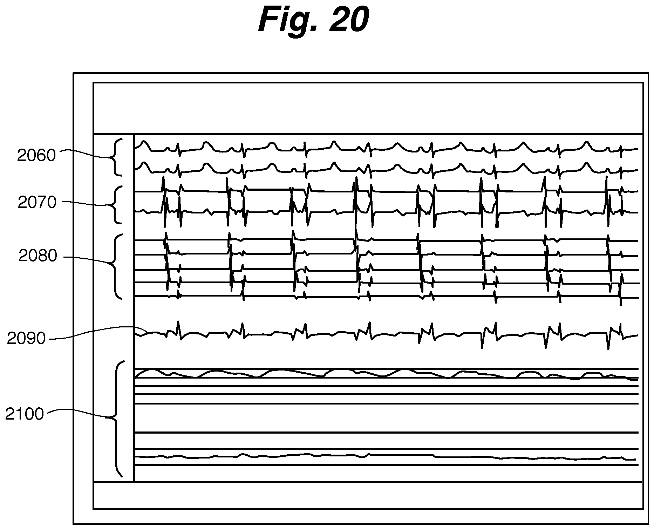

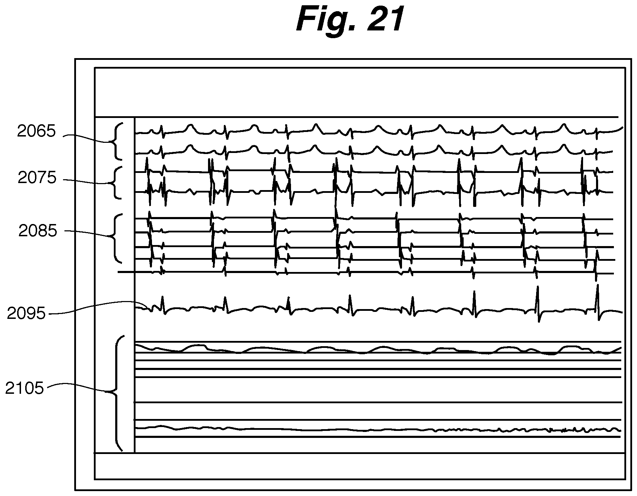

With respect to the step 7 and the exploratory phase of the method, a full set of waveforms is seen in Fig. 20 and Fig. 21. These are representative of the situation with the catheter electrode tip roving in the LA. Trace 2060 and 2065 corresponds to the surface electro gram of the patient, trace 2070 and 2075 corresponds to the His bundle recording. Trace 2080 and 2085 corresponds to the electro gram taken from the coronary vessels while trace 2100 and 2105 are the pressure traces taken from several sensors. Of importance is trace 2090 and 2095 which is the tracing from the needle electrode retracted in its sheath.

## Claims

1. A catheter system (500) for crossing into the left heart from the right heart comprising:
a. a first inner catheter (100), having a sheath (118) and a first working lumen, a first proximal end (102) and having a first distal end (114);
b. a first outer catheter (300) having a first outer catheter working lumen sized to accept said first inner catheter, and having a first outer catheter distal end (314) and having a first outer catheter proximal end, the first outer catheter distal end having a distal tip (316) mechanically engaged with a control knob (306) positioned at the first outer catheter proximal end, whereby when the knob is turned the distal tip moves relative to a longitudinal axis (108) of the distal end a specified distance and angle;
c. a first inner catheter coupler on said first inner catheter;
d. a first outer catheter coupler on said first outer catheter, adapted for mating engagement with said first inner catheter coupler to prevent any relative rotational movement between the coupled first inner catheter and the first outer catheter;
e. a first handle (104) coupled to said first proximal end of said first inner catheter, and having a thumb actuated lever (106) to advance a piercing needle (125) of the first distal end of said first inner catheter into a deployed state;
f. a spring (112) located within said first handle adapted to bias said piecing needle in a retracted state; and
g. an electrical connector (120, 130) located in said first handle to electrically connect to said piercing needle such that the piercing needle is also an electrode, in the retracted state the electrode, along with the first distal end, is moved to measure voltage signals wherein each voltage signal is associated with an anatomical region of the heart.

2. The catheter system of claim 1 further comprising:
a second outer catheter (200) having a second outer catheter working lumen sized to accept said first inner catheter, and having a second outer catheter distal end (214) and having a second outer catheter proximal end, the second outer catheter proximal end defining a torque handle (250); and
a second outer catheter coupler on said second outer catheter, adapted for mating engagement with said first inner catheter coupler to prevent any relative rotational movement between the first inner catheter and the second outer catheter,
wherein the inner catheter may be used with either of the first outer catheter and second outer catheter.

3. The catheter system of claim 1 further comprising a display (133), the display in electronic communication with the electrical connector and piercing needle, the catheter system displaying at the display an electrogram formed by contacting the first distal end of the first inner catheter along with the electrode to a first anatomical region of the heart.

4. The catheter system of claim 3 wherein the electrogram includes an injury current.

5. The catheter system of claim 1 further comprising a guide wire (142), the guide wire having a guide wire proximal end and a guide wire distal end, the first working lumen configured to receive the guide wire therein.

6. The catheter system of claim 3 wherein the piercing needle is a distal portion of a hypo-tube (122) moveable within the sheath, in the deployed state the piercing needle extends distally beyond a distal end of the sheath, in the retracted state the piercing needle is contained entirely within the sheath.

7. The catheter system of claim 6 wherein at least a portion of the hypo-tube defines a plurality of circumferential slits.

8. The catheter system of claim 1 wherein the first outer catheter further comprises a pair of pull wires (357, 359), each pull wire extending from the first outer catheter distal end to a rack positioned within the first outer catheter proximal end, each rack in communication with a pinion gear, the pinion gear in communication with the control knob.

9. The catheter system of claim 1 wherein the torque handle has a longitudinal axis as defined by the second outer catheter working lumen, and a side port being aligned with the longitudinal axis.

## Patentansprüche

1. Kathetersystem (500) zum Kreuzen in das linke Herz von dem rechten Herzen, umfassend:
a. einen ersten Innenkatheter (100), der eine Hülle (118) und ein erstes Arbeitslumen, ein erstes proximales Ende (102) aufweist und ein erstes distales Ende (114) aufweist;
b. einen ersten Außenkatheter (300), der ein erstes Außenkatheterarbeitslumen aufweist, das so bemessen ist, dass es den ersten Innenkatheter aufnimmt, und ein distales Ende (314) des ersten Außenkatheters aufweist, und ein proximales Ende des ersten Außenkatheters aufweist, wobei das distale Ende des ersten Außenkatheters eine distale Spitze (316) aufweist, die mit einem Steuerknopf (306), der an dem proximalen Ende des ersten Außenkatheters positioniert ist, mechanisch in Eingriff steht, wodurch, wenn der Knopf gedreht wird, sich die distale Spitze relativ zu einer Längsachse (108) des distalen Endes um einen spezifizierten Abstand und Winkel bewegt;
c. einen ersten Innenkatheterkoppler an dem ersten Innenkatheter;
d. einen ersten Außenkatheterkoppler auf dem ersten Außenkatheter, der für den Gegeneingriff mit dem ersten Innenkatheterkoppler angepasst ist, um eine relative Drehbewegung zwischen dem gekoppelten ersten Innenkatheter und dem ersten Außenkatheter zu verhindern;
e. einen ersten Griff (104), der mit dem ersten proximalen Ende des ersten Innenkatheters gekoppelt ist und einen Daumenbetätigungshebel (106) aufweist, um eine Durchstechnadel (125) des ersten distalen Endes des ersten Innenkatheters in einen eingesetzten Zustand vorwärtszubewegen;
f. eine Feder (112), die sich innerhalb des ersten Griffs befindet, die angepasst ist, um die Durchstechnadel in einem eingefahrenen Zustand vorzuspannen; und
g. einen elektrischen Verbinder (120, 130), der sich in dem ersten Griff befindet, um mit der Durchstechnadel elektrisch zu verbinden, derart, dass die Durchstechnadel ebenso eine Elektrode ist, wobei in dem eingefahrenen Zustand die Elektrode zusammen mit dem ersten distalen Ende bewegt wird, um Spannungssignale zu messen, wobei jedes Spannungssignal einer anatomischen Region des Herzens zugeordnet ist.

2. Kathetersystem nach Anspruch 1, ferner umfassend:
einen zweiten Außenkatheter (200), der ein zweites Außenkatheterarbeitslumen aufweist, das so bemessen ist, dass es den ersten Innenkatheter aufnimmt, und ein distales Ende (214) des zweiten Außenkatheters aufweist, und ein proximales Ende des zweiten Außenkatheters aufweist, wobei das proximale Ende des zweiten Außenkatheters einen Drehmomentgriff (250) definiert; und
einen zweiten Außenkatheterkoppler auf dem zweiten Außenkatheter, der für den Gegeneingriff mit dem ersten Innenkatheterkoppler ausgelegt ist, um eine relative Drehbewegung zwischen dem ersten Innenkatheter und dem zweiten Außenkatheter zu verhindern,
wobei der Innenkatheter mit einem von dem ersten Außenkatheter und dem zweiten Außenkatheter verwendet werden kann.

3. Kathetersystem nach Anspruch 1, ferner umfassend eine Anzeige (133), wobei die Anzeige in elektronischer Kommunikation mit dem elektrischen Verbinder und der Durchstechnadel ist, wobei das Kathetersystem an der Anzeige ein Elektrogramm anzeigt, das durch Inberührungbringen des ersten distalen Endes des ersten Innenkatheters, zusammen mit der Elektrode, mit einer ersten anatomischen Region des Herzens gebildet wird.

4. Kathetersystem nach Anspruch 3, wobei das Elektrogramm einen Verletzungsstrom einschließt.

5. Kathetersystem nach Anspruch 1, ferner umfassend einen Führungsdraht (142), wobei der Führungsdraht ein proximales Führungsdrahtende und ein distales Führungsdrahtende aufweist, wobei das erste Arbeitslumen konfiguriert ist, um den Führungsdraht darin aufzunehmen.

6. Kathetersystem nach Anspruch 3, wobei die Durchstechnadel ein distaler Abschnitt eines Hypotubes (122) ist, der innerhalb der Hülle beweglich ist, wobei in dem eingesetzten Zustand sich die Durchstechnadel distal über ein distales Ende der Hülle hinaus erstreckt, in dem eingefahrenen Zustand die Durchstechnadel vollständig innerhalb der Hülle enthalten ist.

7. Kathetersystem nach Anspruch 6, wobei mindestens ein Abschnitt des Hypotubes eine Vielzahl von Umfangsschlitzen definiert.

8. Kathetersystem nach Anspruch 1, wobei der erste Außenkatheter ferner ein Paar von Zugdrähten (357, 359) umfasst, wobei sich jeder Zugdraht von dem distalen Ende des ersten Außenkatheters zu einem Rack, das innerhalb des proximalen Endes des ersten Außenkatheters positioniert ist, wobei jedes Rack mit einem Ritzel in Verbindung steht, wobei das Ritzel mit dem Steuerknopf in Verbindung steht.

9. Kathetersystem nach Anspruch 1, wobei der Drehmomentgriff eine Längsachse, wie durch das zweite Außenkatheterarbeitslumen definiert, und eine Seitenöffnung, die mit der Längsachse ausgerichtet ist, aufweist.

## Revendications

1. Système de cathéter (500) pour traverser dans le cœur gauche à partir du cœur droit, comprenant :
a. un premier cathéter interne (100), ayant une gaine (118) et un premier lumen de travail, une première extrémité proximale (102) et ayant une première extrémité distale (114) ;
b. un premier cathéter externe (300) ayant un premier lumen de travail de cathéter externe dimensionné pour accepter ledit premier cathéter interne, et ayant une première extrémité distale de cathéter externe (314) et ayant une première extrémité proximale de cathéter externe, la première extrémité distale de cathéter externe ayant une pointe distale (316) venant en prise mécaniquement avec un bouton de commande (306) positionné au niveau de la première extrémité proximale de cathéter externe, par lequel lorsque le bouton est tourné, l'extrémité distale se déplace par rapport à un axe longitudinal (108) de l'extrémité distale d'une distance et d'un angle spécifiés ;
c. un premier coupleur de cathéter interne sur ledit premier cathéter interne ;
d. un premier coupleur de cathéter externe sur ledit premier cathéter externe, adapté pour venir en prise par accouplement avec ledit premier coupleur de cathéter interne afin d'empêcher tout mouvement de rotation relatif entre le premier cathéter interne couplé et le premier cathéter externe ;
e. une première poignée (104) couplée à ladite première extrémité proximale dudit premier cathéter interne et ayant un levier actionné par le pouce (106) pour avancer une aiguille de perçage (125) de la première extrémité distale dudit premier cathéter interne dans un état déployé ;
f. un ressort (112) situé à l'intérieur de ladite première poignée adapté pour solliciter ladite aiguille de piquage à un état rétracté ; et
g. un connecteur électrique (120,130) situé dans ladite première poignée pour se connecter électriquement à ladite aiguille de perçage de telle sorte que l'aiguille de perçage soit également une électrode, à l'état rétracté l'électrode, conjointement avec la première extrémité distale, est déplacée pour mesurer les signaux de tension dans lequel chaque signal de tension est associé à une région anatomique du coeur.

2. Système de cathéter selon la revendication 1 comprenant en outre :
un second cathéter externe (200) ayant un second lumen de travail de cathéter externe dimensionné pour accepter ledit premier cathéter interne, et ayant une seconde extrémité distale de cathéter externe (214) et ayant une seconde extrémité proximale de cathéter externe, la seconde extrémité proximale de cathéter externe définissant une poignée de couple (250) ; et
un second coupleur de cathéter externe sur ledit second cathéter externe, adapté pour venir en prise par accouplement avec ledit premier coupleur de cathéter interne afin d'empêcher tout mouvement de rotation relatif entre le premier cathéter interne et le second cathéter externe,
dans lequel le cathéter interne peut être utilisé avec l'un parmi le premier cathéter externe et le second cathéter externe.

3. Système de cathéter selon la revendication 1 comprenant en outre un écran (133), l'écran en communication électronique avec le connecteur électrique et l'aiguille de perçage, le système de cathéter affichant au niveau de l'écran un électrogramme formé par la mise en contact de la première extrémité distale du premier cathéter interne avec l'électrode dans une première région anatomique du coeur.

4. Système de cathéter selon la revendication 3, dans lequel l'électrogramme comporte un courant de blessure.

5. Système de cathéter selon la revendication 1 comprenant en outre un fil de guidage (142), le fil de guidage ayant une extrémité proximale de fil de guidage et une extrémité distale de fil de guidage, le premier lumen de travail configuré pour y recevoir le fil de guidage.

6. Système de cathéter selon la revendication 3 dans lequel l'aiguille de perçage est une partie distale d'un hypotube (122) mobile à l'intérieur de la gaine, à l'état déployé l'aiguille de perçage s'étend distalement au-delà d'une extrémité distale de la gaine, à l'état rétracté l'aiguille de perçage est entièrement contenue à l'intérieur de la gaine.

7. Système de cathéter selon la revendication 6 dans lequel au moins une partie de l'hypotube définit une pluralité de fentes circonférentielles.

8. Système de cathéter selon la revendication 1 dans lequel le premier cathéter externe comprend en outre une paire de fils de traction (357, 359), chaque fil de traction s'étendant de la première extrémité distale de cathéter externe à une crémaillère positionnée à l'intérieur de la première extrémité proximale de cathéter externe, chaque crémaillère en communication avec un engrenage à pignon, l'engrenage à pignon en communication avec le bouton de commande.

9. Système de cathéter selon la revendication 1 dans lequel la poignée de couple a un axe longitudinal défini par le second lumen de travail de cathéter externe, et un port latéral étant aligné sur l'axe longitudinal.
